# FASCICULE DE BREVET EUROPEEN

(11) **EP 1 050 530 B1**
(45) Date de publication et mention de la délivrance du brevet: **22.10.2003**
(21) Numéro de dépôt: 00401199.5
(22) Date de dépôt: 02.05.2000
(51) Int. Cl.: C07D 211/86, A61K 31/395, A61P 43/00, C07D 223/08, C07D 409/04, C07D 401/04, C07D 211/74, C07D 211/38, C07D 211/54, C07D 211/72

(54) **1-Aza-2-Alkyl-6-Aryl-Cycloalcanes utiles pour améliorer la mémoire**
1-Aza-2-alkyl-6-aryl-cycloalkanen zur Verbesserung des Gedächtnisses
1-Aza-2-alkyl-6-aryl-cycloalkanes usefull as memory enhancers

(30) Priorité: 03.05.1999 FR 9905600
(43) Date de publication de la demande: 08.11.2000
(73) Titulaire: LES LABORATOIRES SERVIER, 92200 Neuilly sur Seine (FR)
(72) Inventeur: Rault, Sylvain, 14370 Moult (FR); Renault, Olivier, 91120 Palaiseau (FR); Guillon, Jean, 33700 Mérignac (FR); Dallemagne, Patrick, 14260 Saint Georges d'Aunay (FR); Renard, Pierre, 78150 Le Chesnay (FR); Pfeiffer, Bruno, 95320 Saint Leu La Forêt (FR); Lestage, Pierre, 78170 La Celle Saint-Cloud (FR); Lebrun, Marie-Cécile, 92600 Asnières (FR)

(56) Documents cités:
- EP-A- 0 119 087
- ST PHANE CIBLAT ET AL.: "A practical asymmetric synthesis of 2,6-cis-disubstituted piperidines" TETRAHEDRON: ASYMMETRY., vol. 10, - 4 juin 1999 (1999-06-04) pages 2225-2235, XP002128793 ELSEVIER SCIENCE PUBLISHERS, AMSTERDAM., NL ISSN: 0957-4166
- CHEMICAL ABSTRACTS, vol. 106, 1987, Columbus, Ohio, US; abrégé no. 106:5289, page 497
- CHEMICAL ABSTRACTS, vol. 117, 1992, Columbus, Ohio, US; abrégé no. 212413, page 835
- CHEMICAL ABSTRACTS, vol. 128, no. 10, 1998, Columbus, Ohio, US; abrégé no. 114860, page 541
- CHEMICAL ABSTRACTS, vol. 108, 1988, Columbus, Ohio, US; abrégé no. 221623, page 566
- CHEMICAL ABSTRACTS, vol. 120, 1994, Columbus, Ohio, US; abrégé no. 106718, page 1130

## Description

La présente invention concerne de nouveaux 1-aza-2-alkyl-6-aryl-cycloalcanes, leur procédé de préparation, les compositions pharmaceutiques qui les contiennent ainsi que leur utilisation en tant que facilitateurs mnémocognitifs et antalgiques.

Le vieillissement de la population par augmentation de l'espérance de vie a entraîné parallèlement un large accroissement des troubles cognitifs liés au vieillissement cérébral normal ou au vieillissement cérébral pathologique survenant au décours de maladies neurodégénératives telles que, par exemple, la maladie d'Alzheimer.

La plupart des substances utilisées aujourd'hui pour le traitement des troubles cognitifs liés au vieillissement agissent en facilitant les systèmes cholinergiques centraux, soit directement comme c'est le cas des inhibiteurs de l'acétylcholinestérase (tacrine, donepezil) ou des agonistes cholinergiques (nefiracétam), soit indirectement comme dans le cas des nootropes (piracétam, pramiracétam) ou des vasodilatateurs cérébraux (vinpocétine).

Outre leurs propriétés cognitives, les substances agissant directement sur les systèmes cholinergiques centraux ont souvent des propriétés antalgiques, mais également des propriétés hypothermisantes qui peuvent être gênantes.

Il était donc particulièrement intéressant de synthétiser de nouveaux composés capables de s'opposer aux troubles cognitifs liés au vieillissement et/ou d'améliorer les processus cognitifs et pouvant posséder des propriétés antalgiques, mais dépourvus d'activité hypothermisante.

Des 1-aza-2-alkyl-6-aryl-cycloalcanes et 1-aza-2-alkyl-6-aryl-cycloalcènes 4-hydroxy ou 4-oxo- substitués ont déjà été décrits dans la littérature (J. Org. Chem. 1988, 53, 2426 ; Liebigs Ann. Chem. 1986, 11, 1823 ; Synlett 1993, 9, 657 ; Tet. Lett. 1998, 39(3/4), 217), sans qu'aucune activité pharmacologique n'ait été décrite pour ces molécules, ainsi que des 1-aza-6-aryl cycloalcanes 4-hydroxy-ou 4-oxo-substitués, en tant qu'antalgiques (EP 0119087).

Plus spécifiquement, la présente invention concerne les composés de formule (I) : 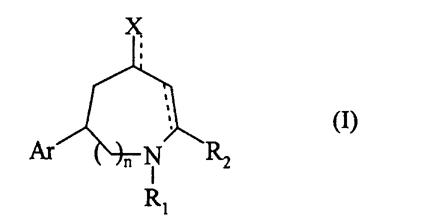 dans laquelle :
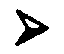 n représente 0 ou 1,
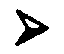 R₁ représente un atome d'hydrogène,
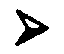 R₂ représente un groupement alkyle (C₁-C₆) linéaire ou ramifié,
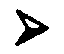 X représente un atome d'oxygène ou un groupement OR₃,
   * R₃ représente un atome d'hydrogène,
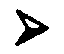 ---- représente une liaison simple ou double, étant entendu que la valence des atomes est respectée,
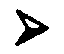 Ar représente un groupement aryle ou un groupement hétéroaryle,
leurs isomères ainsi que leurs sels d'addition à un acide pharmaceutiquement acceptable,
à la condition que les composés de formule (I) soient différents :
- de la 6-méthyl-2-phényl-2,3-dihydro-4-pyridinone,
- de la 2-méthyl-6-phényl-4-pipéridinone,
- des N-benzyl-2-(R'₂)-6-phényl-4-pipéridinones dans lesquelles R'₂ représente le groupement méthyle, éthyle, propyle ou isopropyle,
- et des 2-(R"₂)-6-phényl-4-pipéridinols dans lesquels R"₂ représente le groupement isopropyle ou butyle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique, etc...

Par groupement aryle, on entend phényle éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié).

Par groupement hétéroaryle, on entend thiényle ou pyridyle éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié).

Les composés préférés de formule (I) sont ceux pour lesquels n représente 0.

L'invention s'étend également au procédé de préparation des composés de formule (I), caractérisé en ce que l'on met en réaction un composé de formule (II) : 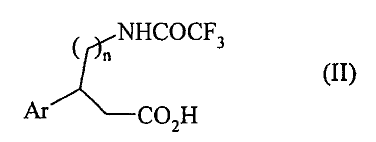 dans laquelle Ar et n ont la même signification que dans la formule (I),
avec du chlorure de thionyle, pour conduire au chlorure d'acide correspondant que l'on fait réagir avec un composé de formule (III) : 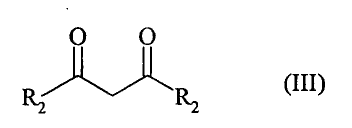 dans laquelle R₂ a la même signification que dans la formule (I),
en présence de triiodure de samarium,
pour conduire après déprotection par un acide HA au composé de formule (IV) : 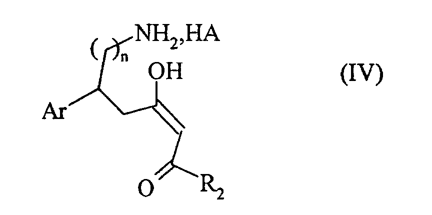 dans laquelle Ar, n et R₂ ont la même signification que précédemment, et HA représente un acide donneur de proton, composé de formule (IV), que l'on fait ensuite réagir en milieu basique pour conduire au composé de formule (la), cas particulier des composés de formule (I) : 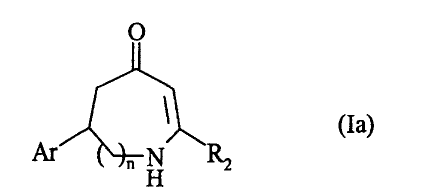 dans laquelle Ar, n et R₂ ont la même signification que précédemment,
composé de formule (Ia) que l'on transforme, si on le désire,
- soit par réduction partielle à l'aide d'un agent réducteur approprié, suivie, lorsqu'on le souhaite, d'une alkylation, acylation ou estérification de la fonction hydroxy, pour conduire au composé de formule (Ic), cas particulier des composés de formule (I) : 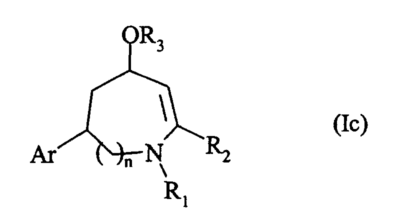
dans laquelle Ar, n et R₂ ont la même signification que précédemment, et R₁ et R₃ ont la même signification que dans la formule (I),
- soit par réduction totale à l'aide d'un agent réducteur approprié, pour conduire au composé de formule (Id), cas particulier des composés de formule (I) : 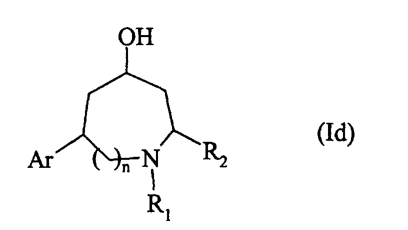
dans laquelle Ar, n, R₁ et R₂ ont la même signification que précédemment, composé de formule (Id) que l'on fait réagir, si on le souhaite,
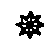 par une réaction d'oxydation à l'aide d'un agent oxydant approprié, pour conduire au composé de formule (If), cas particulier des composés de formule (I): 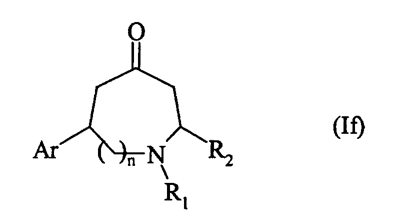 dans laquelle Ar, n, R₁ et R₂ ont la même signification que précédemment,
   composés de formule (Ia), (Ic), (Id), ou (If), qui constituent l'ensemble des composés de formule (I), que l'on purifie, le cas échéant, selon une technique classique de purification, dont on sépare, si on le souhaite, les isomères selon une technique classique de séparation, et que l'on transforme, si on le souhaite, en leurs sels d'addition à un acide pharmaceutiquement acceptable.

Le composé de formule (II) est obtenu à partir du composé de formule (VI) :

Ar-CHO (VI)

dans laquelle Ar a la même signification que précédemment, que l'on transforme en un composé de formule (VII) : 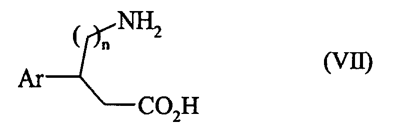 dans laquelle Ar et n ont la même signification que précédemment,
- lorsque n est égal à 0, selon le procédé décrit par W.M. Rodionow et E.Th. Malewinskaya dans Ber. 1926, 59, 2952 et T.B. Johnson, J.E. Livak dans J. Am. Chem. Soc. 1936, 58, 299
- lorsque n est égal à 1, selon le procédé décrit par Keberle dans le brevet CH 449046,
composé de formule (VII) que l'on dédouble éventuellement (lorsqu'on souhaite obtenir le composé de formule (I) sous sa forme énantiomériquement pure) selon une technique classique de dédoublement, avant de le transformer en composé de formule (II) par réaction avec l'anhydride trifluoroacétique.

Les composés de la présente invention, outre le fait qu'ils soient nouveaux, présentent des propriétés facilitatrices des processus cognitifs et antalgiques qui les rendent utiles dans le traitement des déficits cognitifs associés au vieillissement cérébral et aux pathologies neurodégénératives telles que la maladie d'Alzheimer, la maladie de Parkinson, la maladie de Pick, la maladie de Korsakoff, les démences frontales et sous-corticales et dans le traitement de la douleur.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéfiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention et ne la limitent en aucune façon. Par DMSO, on entend diméthylsulfoxyde.

Par composé de configuration relative (2R*, 4S*, 6R*), on entend mélange racémique des composés de configurations absolues (2R, 4S, 6R) et (2S, 4R, 6S).

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse).

### EXEMPLE 1 : (±)-2-(3-Chlorophényl)-6-méthyl-2,3-dihydro-4(1H)-pyridinone

### Stade A : (±)-3-(3-Chlorophényl)-β-alanine

Le produit attendu est obtenu selon le procédé décrit dans Ber. 1926, 59, 2952 et JACS 1936, 58, 299, à partir de 3-chlorobenzaldéhyde.

### Stade B : (±)-3-(3-Chlorophényl)-N-trifluoroacétyl-β-alanine

A 10 mmoles du composé obtenu dans le stade précédent, est ajouté de l'anhydride trifluoroacétique (11 mmoles). Après 30 mn d'agitation, le solvant et le réactif excédentaire sont évaporés sous pression réduite pour conduire au produit attendu.

### Stade C : Chlorure de l'acide (±)-3-(3-chlorophényl)-3-trifluoroacétylamino propionique

A 10 mmoles du composé décrit dans le stade précédent sont ajoutés 20 ml de chlorure de thionyle, puis le mélange est porté à reflux. Après 80 mn, le chlorure de thionyle est évaporé, puis le résidu est repris dans 50 ml d'éther de pétrole. Le précipité obtenu est filtré et lavé par de l'éther de pétrole.

### Stade D : Chlorure de (±)-1-(3-chlorophényl)-3-hydroxy-5-oxo-3-hexénylammonium

11 mmoles de samarium et 16,5 mmoles d'iode sont mis en réaction dans l'acétonitrile sec. Après 12 h d'agitation à température ambiante, la solution est refroidie à 0°C, puis le chlorure d'acide obtenu dans le stade précédent (10 mmoles) et 11 mmoles de pentane-2,4-dione sont ajoutés. Le mélange réactionnel est laissé sous agitation pendant 6 h à 0°C. 50 ml d'une solution d'acide chlorhydrique (6N) sont additionnés, l'acétonitrile est évaporé, puis 50 ml d'acide chlorhydrique (6N) sont ajoutés. La phase aqueuse est extraite par de l'éther. Les phases organiques rassemblées sont lavées par une solution de bicarbonate de sodium saturée puis par une solution de thiosulfate de sodium saturée, puis elles sont séchées et évaporées. Le résidu solide est repris dans un mélange 50/50 d'acide chlorhydrique et d'éthanol, la suspension est portée à reflux pendant 12 h, puis évaporée pour conduire au produit attendu sous forme de cristaux jaunes.

Point de fusion : 97°C
- IR (KBr):: Bande OH à 3415 cm⁻¹
Bande NH₃⁺ entre 3255 et 2500 cm⁻¹
Bande CO à 1725 cm⁻¹

### Stade E : (±)-2-(3-Chlorophényl)-6-méthyl-2,3-dihydro-4(1H)-pyridinone

A 10 mmoles du composé obtenu dans le stade précédent en solution dans le dichlorométane est introduite au goutte à goutte une solution d'ammoniaque concentrée, jusqu'à ce que le mélange soit à pH ≥ 10.

La phase aqueuse est extraite par du dichlorométhane puis les phases organiques rassemblées sont séchées et évaporées. Le résidu huileux est repris par 50 ml d'éther. Par filtration du précipité obtenu, on obtient le produit attendu sous la forme de cristaux de couleur beige.

Point de fusion : 163°C

| Microanalyse élémentaire : | | | |
|---|---|---|---|
| | %C | %H | %N |
| calculé | 65,02 | 5,91 | 6,32 |
| trouvé | 64,89 | 6,02 | 6,25 |

### EXEMPLE 2 : (±)-N-Méthyl-2-(3-chlorophényl)-6-méthyl-2,3-dihydro-4-(1H)-pyridinone

A 10 mmoles du composé décrit dans l'exemple 1 en solution dans le tétrahydrofurane est additionnée, au goutte à goutte à -78°C, une solution 1,6M de n-butyllithium dans l'hexane (11 mmoles). Après ½ h d'agitation à -78°C, 11 mmoles d'iodométhane sont additionnées. L'agitation est maintenue à -78°C pendant encore 30 mn, puis 1 h à température ambiante. Après hydrolyse par ajout d'une solution saturée d'hydrogénocarbonate de sodium, la phase aqueuse est extraite au dichlorométhane. Les phases organiques rassemblées sont lavées, séchées et évaporées pour conduire au produit attendu sous la forme de cristaux oranges.
Point de fusion : 82°C

| Microanalyse élémentaire : | | | |
|---|---|---|---|
| | %C | %H | %N |
| calculé | 57,39 | 6,23 | 3,94 |
| trouvé | 57,82 | 6,21 | 3,78 |

### EXEMPLE 3 : (2R*,4S*,6R*)-N-Méthyl-2-(3-chlorophényl)-6-méthyl-4-pipéridinol, fumarate

### Stade A : (2R*,4S*,6R*)-N-Méthyl-2-(3-chlorophényl)-6-méthyl-4-pipéridinol

A 10 mmoles du composé décrit dans l'exemple 2 en solution à 0°C dans l'éthanol sont ajoutées 80 mmoles de borohydrure de sodium. Après 12 h d'agitation à température ambiante, l'éthanol est évaporé et le résidu blanc est repris par du dichlorométhane. La phase organique est lavée par une solution d'acide chlorhydrique 2N puis les phases aqueuses rassemblées sont saturées en chlorure de sodium, alcalinisées par une solution d'ammoniaque concentrée puis extraites par le dichlorométhane. Les phases organiques rassemblées sont séchées puis évaporées pour conduire au produit attendu sous la forme d'une huile incolore.

### Stade B : (2R*,4S*,6R*)-N-Méthyl-2-(3-chlorophényl)-6-méthyl-4-pipéridinol, fumarate

A 10 mmoles du composé obtenu dans le stade précédent en solution dans l'isopropanol sont ajoutées 11 mmoles d'acide fumarique. Après 1 h d'agitation à température ambiante, les solvants sont évaporés pour conduire au produit attendu sous la forme de cristaux blancs.
Point de fusion : 182°C

### EXEMPLE 4 : (2R*,4S*,6R*)-N-Méthyl-2-(3-chlorophényl)-4-méthoxy-6-méthylpipéridine

A 11 mmoles d'hydrure de sodium en suspension dans le tétrahydrofurane sont ajoutées, à 0°C, 10 mmoles du composé décrit dans l'exemple 3. Après ½ h d'agitation à 0°C sont additionnées 100 mmoles de iodométhane. L'agitation est ensuite maintenue à température ambiante pendant 2 jours, puis la solution est hydrolysée par ajout d'une solution 1N d'acide chlorhydrique. La phase aqueuse est extraite au dichlorométhane, puis les phases organiques rassemblées sont lavées, séchées et évaporées pour conduire au produit attendu sous la forme d'une huile jaune pâle.

### EXEMPLE 5 : (2R*,4S*)-N-Méthyl-2-(3-chlorophényl)-6-méthyl-1,2,3,4-tétrahydro-4-pyridinol

A 10 mmoles du composé décrit dans l'exemple 2 en solution dans l'éthanol sont ajoutées 10 mmoles de CeCl₃ heptahydrate, puis, à 0°C et par portions, 10 mmoles de borohydrure de sodium. Après ½ h d'agitation à 0°C puis 1 h à température ambiante, l'éthanol est évaporé et le résidu blanc est repris par du dichlorométhane. La phase organique est lavée par une solution d'acide chlorhydrique 2N puis les phases aqueuses rassemblées sont saturées en chlorure de sodium, basifiées par une solution d'ammoniaque concentrée puis extraites par le dichlorométhane. Les phases organiques rassemblées sont séchées puis évaporées pour conduire au produit attendu.

### EXEMPLE 6 : (2R*,4S*,6R*)-2-(3-Chlorophényl)-6-méthyl-4-pipéridinol, chlorhydrate

### Stade A : (2R*,4S*,6R*)-2-(3-Chlorophényl)-6-méthyl-4-pipéridinol

Le produit attendu est obtenu selon le procédé décrit dans le stade A de l'exemple 3 à partir du composé décrit dans l'exemple 1.

### Stade B : (2R*,4S*,6R*)-2-(3-Chlorophényl)-6-méthyl-4-pipéridinol, chlorhydrate

A 10 mmoles du composé obtenu dans le stade précédent en solution dans l'isopropanol sont ajoutés 11 ml d'une solution d'éther chlorhydrique 1M. Après 1 h d'agitation à température ambiante, les solvants sont évaporés pour conduire au produit attendu sous la forme de cristaux de couleur beige.
Point de fusion : 248°C

| Microanalyse élémentaire : | | | |
|---|---|---|---|
| | %C | %H | %N |
| calculé | 45,82 | 6,53 | 5,34 |
| trouvé | 45,54 | 6,62 | 5,41 |

### EXEMPLE 7 : (2R*,4S*,6R*)-2-(3-Chlorophényl)-6-méthyl-4-pipéridinol, oxalate

A 10 mmoles du composé obtenu dans le stade A de l'exemple 6 en solution dans l'isopropanol sont ajoutées 11 mmoles d'acide oxalique. Après 1 h d'agitation à température ambiante, les solvants sont évaporés pour conduire au produit attendu sous forme de cristaux.
Point de fusion : 224°C

### EXEMPLE 8 : (2R*,4S*,6R*)-N-Trifluoroacétyl-2-(3-chlorophényl)-6-méthyl-4-pipéridinol, chlorhydrate

### Stade A : (±)-N-Trifluoroacétyl-2-(3-chlorophényl)-6-méthyl-2,3-dihydro-4(1H)-pyridinone

Le produit attendu est obtenu selon le procédé décrit dans l'exemple 2 à partir du composé décrit dans l'exemple 1, en remplaçant l'iodométhane par le chlorure de trifluoroacétyle.

### Stade B : (2R*,4S*,6R*)-N-Trifluoroacétyl-2-(3-chlorophényl)-6-méthyl-4-pipéridinol, chlorhydrate

Le produit attendu est obtenu selon le procédé décrit dans l'exemple 6 à partir du composé obtenu dans le stade précédent.

### EXEMPLE 9 : (2R *,4S*)-2-(3-Chlorophényl)-6-méthyl-1,2,3,4-tétrahydro-4-pyridinol

Le produit attendu est obtenu selon le procédé décrit dans l'exemple 5 à partir du composé décrit dans l'exemple 1.

### EXEMPLE 10 : (2R*,4S*,6R*)-2-(3-Chlorophényl)-6-méthyl-4-oxo-3,4-dihydro-1(2H)-pyridinecarboxylate de benzyle

Le produit attendu est obtenu selon le procédé décrit dans l'exemple 2 à partir du composé décrit dans l'exemple 1, en remplaçant l'iodométhane par le chloroformiate de benzyle.

### EXEMPLE 11 : (2R*,4S*)-2-(3-Chlorophényl)-4-hydroxy-6-méthyl-3,4-dihydro-1(2H)-pyridinecarboxylate de benzyle

Le produit attendu est obtenu selon le procédé décrit dans l'exemple 5 à partir du composé décrit dans l'exemple 10.

### EXEMPLE 12 : (2R*,6R*)-2-(3-Chlorophényl)-6-méthyl-4-pipéridinone, oxalate

### Stade A : (2R*,6R*)-2-(3-Chlorophényl)-6-méthyl-4-pipéridinone

A 10 mmoles du composé décrit dans le stade A de l'exemple 6 en solution dans l'acétone à O°C sont ajoutées au goutte à goutte 20 mmoles d'acide orthophosphorique, puis 20 mmoles d'anhydride chromique.

Après 1 h d'agitation à O°C, puis 12 h d'agitation à température ambiante, l'acétone est évaporée, le résidu est repris par 20 ml de glace puis alcalinisé à pH>10 par une solution d'ammoniaque à 28 %. La solution est ensuite extraite par le dichlorométhane. Les phases organiques rassemblées sont séchées puis évaporées pour conduire au produit attendu.

### Stade B : (2R*,6R*)-2-(3-Chlorophényl)-6-méthyl-4-pipéridinone, oxalate

Le produit attendu est obtenu selon le procédé décrit dans l'exemple 7 à partir du composé obtenu dans le stade précédent.
Point de fusion : 183°C

### EXEMPLE 13 : (2R*,4R*,6R*)-4-Chloro-2-(3-chlorophényl)-6-méthyl-pipéridine, fumarate

### Stade A : (2R*,4R*,6R*)-4-Chloro-2-(3-chlorophényl)-6-méthyl-pipéridine

A 10 mmoles du composé décrit dans le stade A de l'exemple 12 en solution dans le chloroforme sont additionnées 20 mmoles de triéthylamine suivies de 20 mmoles de chlorure de thionyle. Le mélange réactionnel est chauffé au reflux pendant 2 h. Après retour à température ambiante, la phase organique est lavée, les phases aqueuses rassemblées sont alcalinisées par ajout d'une solution d'ammoniaque puis extraite au dichlorométhane, et les phases organiques rassemblées sont lavées, séchées et évaporées pour conduire au produit attendu.

### Stade B : (2R*,4R*,6R*)-4-Chloro-2-(3-chlorophényl)-6-méthyl-pipéridine, fumarate

Le produit attendu est obtenu selon le procédé décrit dans le stade B de l'exemple 3 à partir du composé obtenu dans le stade précédent.
Point de fusion : 203°C

### EXEMPLE 14 : (2R *,4S*,6R*)-2-(3-Chlorophényl)-6-méthyl-4-phénylthio-pipéridine, fumarate

### Stade A : (2R*,4S*,6R*)-2-(3-Chlorophényl)-6-méthyl-4-phénylthio-pipéridine

A 30 mmoles d'hydrure de sodium en suspension dans le diméthylformamide sont ajoutées, à 0°C, 13 mmoles de thiophénol puis, après 30 mn d'agitation, 10 mmoles du composé décrit dans le stade A de l'exemple 13. Le mélange réactionnel est ensuite chauffé au reflux pendant 2 h, puis la solution est hydrolysée par ajout d'une solution 1N d'acide chlorhydrique. La phase aqueuse est lavée à l'éther puis alcalinisée par ajout d'une solution d'ammoniaque et extraite à l'éther. Les phases organiques rassemblées sont lavées à l'eau, séchées puis évaporées pour conduire au produit attendu.

### Stade B : (2R*,4S*,6R*)-2-(3-Chlorophényl)-6-méthyl-4-phénylthio-pipéridine, fumarate

Le produit attendu est obtenu selon le procédé décrit dans le stade B de l'exemple 3 à partir du composé obtenu dans le stade précédent.
Point de fusion : 181°C

### EXEMPLE 15 : (±)-2-(3-Chlorophényl)-6-méthyl-2,3-dihydro-4(1H)-pyridinoneoxime

A 10 mmoles du composé décrit dans l'exemple 1 en solution dans l'éthanol sont additionnées 40 mmoles de chlorhydrate d'hydroxylamine et 40 mmoles d'acétate de sodium, puis le mélange réactionnel est porté au reflux pendant 2 heures. Après hydrolyse, la phase aqueuse est extraite au dichlorométhane, puis les phases organiques rassemblées sont lavées, séchées et évaporées pour conduire au produit attendu sous la forme de cristaux oranges.
Point de fusion : 88°C (Mélange Z/E 20/80)

| Microanalyse élémentaire : | | | |
|---|---|---|---|
| | %C | %H | %N |
| calculé | 60,89 | 5,54 | 11,83 |
| trouvé | 60,95 | 5,62 | 11,15 |

### EXEMPLE 16 : (2R)-2-(3-Chlorophényl)-6-méthyl-2,3-dihydro-4-(1H)-pyridinone

### Stade A : (±)-N-Phénylacétyl-3-(3-chlorophényl)-β-alanine

A 10 mmoles de (±)-3-(3-chlorophényl)-β-alanine décrite dans le stade A de l'exemple 1 en solution dans un mélange eau/acétone 3/1 sont ajoutées 24 mmoles de triéthylamine, puis à -5°C, 13 mmoles de chlorure de phénylacétyle. Après 2 h d'agitation à -5°C puis 3 h d'agitation à température ambiante, la solution est filtrée, l'acétone est évaporée, la phase aqueuse est lavée par de l'éther puis acidifiée à pH=1 et extraite par l'acétate d'éthyle. Les phases organiques rassemblées sont séchées puis évaporées. Le résidu obtenu est lavé par l'hexane, précipité dans un minimum d'éther puis filtré pour conduire au produit attendu.

### Stade B : (3R)-3-(3-Chlorophényl)-β-alanine, chlorhydrate

A 10 mmoles du composé décrit dans le stade précédent dans une solution de bicarbonate de sodium saturée à 37°C, on introduit, après ajustement du pH entre 7,4 et 7,6 à l'aide d'acide chlorhydrique concentré, de la pénicilline amidase (20 mg). Après 24 h d'agitation à pH compris entre 7,5 et 8, le mélange est amené à pH=1 avec de l'acide chlorhydrique. La phase aqueuse est lavée par de l'éther puis évaporée. Le solide blanc obtenu est repris par de l'éthanol puis filtré. Le filtrat est ensuite évaporé pour conduire au produit attendu.

### Stade C : (2R)-2-(3-Chlorophényl)-6-méthyl-2,3-dihydro-4-(1H)-pyridinone

Le produit attendu est obtenu selon le procédé décrit dans les stades B à E de l'exemple 1 à partir du composé décrit dans le stade précédent.
Pouvoir rotatoire : [α]²⁰_{D} = -176,92 (c=0,0039 ; CH₃OH)

### EXEMPLE 17 : (2R,4S,6R)-2-(3-Chlorophényl)-6-méthyl-4-pipéridinol, oxalate

### Stade A : (2R,4S,6R)-2-(3-Chlorophényl)-6-méthyl-4-pipéridinol

Le produit attendu est obtenu selon le procédé décrit dans le stade A de l'exemple 3 à partir du composé décrit dans l'exemple 16.

### Stade B : (2R,4S,6R)-2-(3-Chlorophényl)-6-méthyl-4-pipéridinol, oxalate

Le produit attendu est obtenu selon le procédé décrit dans l'exemple 7 à partir du composé obtenu dans le stade précédent.
Pouvoir rotatoire : [α]²⁰_{D} = -9,58 (c=0,0024 ; H₂O)

### EXEMPLE 18 : (2S)-2-(3-Chlorophényl)-6-méthyl-2,3-dihydro-4-(1H)-pyridinone

### Stade A : (3S)-3-(3-Chlorophényl)-β-alanine, chlorhydrate

A 10 mmoles du composé décrit dans le stade A de l'exemple 16 dans une solution de bicarbonate de sodium saturée à 37°C, on introduit, après ajustement du pH entre 7,4 et 7,6 à l'aide d'acide chlorhydrique concentré, de la pénicilline amidase (20 mg). Après 24 h d'agitation à pH compris entre 7,5 et 8, le mélange est amené à pH=1 avec de l'acide chlorhydrique. La phase aqueuse est extraite par de l'éther puis les phases organiques obtenues sont rassemblées, séchées et évaporées. Le résidu est repirs dans l'hexane et précipité par un minimum d'éther. Après filtration, le solide obtenu est chauffé à 50°C pendant 24 heures dans l'acide chlorhydrique 2N pour conduire au produit attendu.

### Stade B : (2S)-2-(3-Chlorophényl)-6-méthyl-2,3-dihydro-4-(1H)-pyridinone

Le produit attendu est obtenu selon le procédé décrit dans les stades B à E de l'exemple 1 à partir du composé décrit dans le stade précédent.
Pouvoir rotatoire : [α]²⁰_{D} = + 179,7 (c=0,080 ; CH₃OH)

### EXEMPLE 19 : (2S,4R,6S)-2-(3-Chlorophényl)-6-méthyl-4-pipéridinol, fumarate

### Stade A : (2S,4R,6S)-2-(3-Chlorophényl)-6-méthyl-4-pipéridinol

Le produit attendu est obtenu selon le procédé décrit dans le stade A de l'exemple 3 à partir du composé décrit dans l'exemple 18.

### Stade B : (2S,4R,6S)-2-(3-Chlorophényl)-6-méthyl-4-pipéridinol, fumarate

Le produit attendu est obtenu selon le procédé décrit dans le stade B de l'exemple 3 à partir du composé obtenu dans le stade précédent.
Pouvoir rotatoire : [α]²⁰_{D} = +9,8 (c=0,067 ; DMSO)

### EXEMPLE 20 : (2S,6S)-2-(3-Chlorophényl)-6-méthyl-4-pipéridinone, oxalate

Le produit attendu est obtenu selon le procédé décrit dans l'exemple 12 à partir du composé décrit dans le stade A de l'exemple 19.

Point de fusion : 184°C

Pouvoir rotatoire : [α]²⁰_{D} = -24,2 (c=0,065 ; DMSO)

### EXEMPLE 21 : (2R*,4S*,6R*)-2-Phényl-6-méthyl-4-pipéridinol, chlorhydrate

### Stade A : (±)-2-Phényl-6-méthyl-2,3-dihydro-1H-4-pyridinone

Le produit attendu est obtenu selon le procédé décrit dans l'exemple 1 à partir du benzaldéhyde.
Cristaux jaunes.
Point de fusion : 156°C

| Microanalyse élémentaire : | | | |
|---|---|---|---|
| | %C | %H | %N |
| calculé | 76,97 | 7,00 | 7,18 |
| trouvé | 76,69 | 7,11 | 7,39 |

### Stade B : (2R*,4S*,6R*)-2-Phényl-6-méthyl-4-pipéridinol

Le produit attendu est obtenu selon le procédé décrit dans le stade A de l'exemple 3 à partir du composé décrit dans le stade précédent.

### Stade C : (2R*,4S*,6R*)-2-Phényl-6-méthyl-4-pipéridinol, chlorhydrate

Le produit attendu est obtenu selon le procédé décrit dans le stade B de l'exemple 6 à partir du composé décrit dans le stade précédent.
Cristaux beiges.
Point de fusion : 236°C

| Microanalyse élémentaire : | | | |
|---|---|---|---|
| | %C | %H | %N |
| calculé | 63,29 | 7,97 | 6,15 |
| trouvé | 62,95 | 8,09 | 5,99 |

### EXEMPLE 22 : (2R*,4S*,6R*)-2-Phényl-6-méthyl-4-pipéridinol, oxalate

Le produit attendu est obtenu selon le procédé décrit dans l'exemple 7, à partir du composé décrit dans le stade B de l'exemple 21.

### EXEMPLE 23 : (±)-2-(2-Chlorophényl)-6-méthyl-2,3-dihydro-4(1H)-pyridinone

Le produit attendu est obtenu selon le procédé décrit dans l'exemple 1, à partir de 2-chlorobenzaldéhyde.
Cristaux beiges.
Point de fusion : 98°C

| Microanalyse élémentaire : | | | |
|---|---|---|---|
| | %C | %H | %N |
| calculé | 65,02 | 5,91 | 6,32 |
| trouvé | 65,20 | 5,93 | 6,32 |

### EXEMPLE 24 : (±)-2-(4-Chlorophényl)-6-méthyl-2,3-dihydro-4(1H)-pyridinone

Le produit attendu est obtenu selon le procédé décrit dans l'exemple 1, à partir de 4-chlorobenzaldéhyde.
Cristaux beiges.
Point de fusion : 104°C

| Microanalyse élémentaire : | | | |
|---|---|---|---|
| | %C | %H | %N |
| calculé | 65,02 | 5,91 | 6,32 |
| trouvé | 64,90 | 5,92 | 6,31 |

### EXEMPLE 25 : (±)-6-(4-Chlorophényl)-2-méthyl-1,5,6,7-tétrahydro-4H-azépin-4-one

L'acide 4-amino-3-(4-chlorophényl) butyrique (baclofène) est soumis aux opérations décrites dans les stades B à E de l'exemple 1, pour conduire au produit attendu sous forme de cristaux crème.
Point de fusion : 118°C

| Microanalyse élémentaire : | | | |
|---|---|---|---|
| | %C | %H | %N |
| calculé | 66,24 | 5,99 | 5,84 |
| trouvé | 66,25 | 5,84 | 6,17 |

### EXEMPLE 26 : (±)-2-(3,4-Dichlorophényl)-6-méthyl-2,3-dihydro-4(1H)-pyridinone

Le produit attendu est obtenu selon le procédé décrit dans l'exemple 1, à partir de 3,4-dichlorobenzaldéhyde.
Cristaux beiges.
Point de fusion : 163°C

| Microanalyse élémentaire : | | | |
|---|---|---|---|
| | %C | %H | %N |
| calculé | 56,27 | 4,33 | 5,47 |
| trouvé | 56,27 | 4,33 | 5,45 |

### EXEMPLE 27 : (±)-2-(3-Méthoxyphényl)-6-méthyl-2,3-dihydro-4(1H)-pyridinone

Le produit attendu est obtenu selon le procédé décrit dans l'exemple 1, à partir de 3-méthoxybenzaldéhyde.
Cristaux beiges.
Point de fusion : 98°C

| Microanalyse élémentaire : | | | |
|---|---|---|---|
| | %C | %H | %N |
| calculé | 71,87 | 6,96 | 6,45 |
| trouvé | 71,82 | 6,66 | 6,72 |

### EXEMPLE 28 : (+)-6-Méthyl-2-(2-thiényl)-23-dihydro-4(1H)-pyridinone

Le produit attendu est obtenu selon le procédé décrit dans l'exemple 1, à partir de thiophène-2-carboxaldéhyde.
Cristaux beiges.
Point de fusion : 114°C

| Microanalyse élémentaire : | | | |
|---|---|---|---|
| | % C | % H | % N |
| calculé | 62,15 | 5,74 | 7,25 |
| trouvé | 62,30 | 5,81 | 7,08 |

### EXEMPLE 29 : (2R*,4S*,6R*)-6-Méthyl-2-(2-thiényl)-4-pipéridinol, chlorhydrate

Le produit attendu est obtenu selon le procédé décrit dans l'exemple 6, à partir du composé décrit dans l'exemple 28.
Cristaux beiges.
Point de fusion : 248°C

| Microanalyse élémentaire : | | | |
|---|---|---|---|
| | %C | %H | %N |
| calculé | 51,38 | 6,90 | 5,99 |
| trouvé | 51,16 | 7,01 | 5,79 |

### EXEMPLE 30 : (±)-6-Méthyl-2-(3-thiényl)-2,3-dihydro-4(1H)-pyridinone

Le produit attendu est obtenu selon le procédé décrit dans l'exemple 1, à partir de thiophène-3-carboxaldéhyde.
Cristaux bruns.
Point de fusion : 103°C

| Microanalyse élémentaire : | | | |
|---|---|---|---|
| | %C | %H | %N |
| calculé | 62,15 | 5,74 | 7,25 |
| trouvé | 62,27 | 5,82 | 7,20 |

### EXEMPLE 31 : (2R*,4S*,6R*)-6-Méthyl-2-(3-thiényl)-4-pipéridinol, chlorhydrate

### Stade A : (2R*,4S*,6R*)-6-Méthyl-2-(3-thiényl)-4-pipéridinol

Le produit attendu est obtenu selon le procédé décrit dans le stade A de l'exemple 3 à partir du composé décrit dans l'exemple 30.

### Stade B : (2R*,4S*,6R*)-6-Méthyl-2-(3-thiényl)-4-pipéridinol, chlorhydrate

Le produit attendu est obtenu selon le procédé décrit dans le stade B de l'exemple 6 à partir du composé décrit dans le stade précédent.
Cristaux beiges.
Point de fusion: 241°C

| Microanalyse élémentaire : | | | |
|---|---|---|---|
| | %C | %H | %N |
| calculé | 51,38 | 6,90 | 5,99 |
| trouvé | 51,26 | 6,81 | 5,81 |

### EXEMPLE 32 : (2R*,4S*,6R*)-6-Méthyl-2-(3-thiényl)-4-pipéridinol, oxalate

Le produit attendu est obtenu selon le procédé décrit dans l'exemple 7 à partir du composé décrit dans le stade A de l'exemple 31.
Point de fusion : 246°C

### EXEMPLE 33 : (2R*,6R*)-6-Méthyl-2-(3-thiényl)-4-pipéridinone, oxalate

Le produit attendu est obtenu selon le procédé décrit dans l'exemple 12 à partir du composé décrit dans le stade A de l'exemple 31.
Point de fusion : 184°C

### EXEMPLE 34 : (±)-2-(4-Bromo-2-thiényl)-6-méthyl-2,3-dihydro-4(1H)-pyridinone

Le produit attendu est obtenu selon le procédé décrit dans l'exemple 1 à partir de 4-bromothiophène-2-carboxaldéhyde.
Cristaux beiges.
Point de fusion : 170°C

| Microanalyse élémentaire : | | | |
|---|---|---|---|
| | % C | % H | % N |
| calculé | 44,13 | 3,70 | 5,15 |
| trouvé | 44,01 | 3,78 | 5,06 |

### EXEMPLE 35 : (±)-2-(4-Chloro-2-thiényl)-6-méthyl-2,3-dihydro-4(1H)-pyridinone

Le produit attendu est obtenu selon le procédé décrit dans l'exemple 1 à partir de 4-chlorothiophène-2-carboxaldéhyde.
Cristaux beiges.
Point de fusion : 141°C

| Microanalyse élémentaire : | | | |
|---|---|---|---|
| | %C | %H | %N |
| calculé | 52,75 | 4,43 | 6,15 |
| trouvé | 52,52 | 4,49 | 6,01 |

### EXEMPLE 36 : (±)-6-Méthyl-2-(3-pyridyl)-2,3-dihydro-4(1H)-pyridinone

Le produit attendu est obtenu selon le procédé décrit dans l'exemple 1 à partir de nicotinaldéhyde.
Cristaux jaunes.
Point de fusion : 141°C

| Microanalyse élémentaire : | | | |
|---|---|---|---|
| | %C | %H | %N |
| calculé | 52,75 | 4,43 | 6,15 |
| trouvé | 52,52 | 4,49 | 6,01 |

### EXEMPLE 37 (2R*,4S*,6R*)-6-Méthyl-2-(3-pyridyl)-4-pipéridinol, dioxalate

### Stade A : (2R*,4S*,6R*)-6-Méthyl-2-(3-pyridyl)-4-pipéridinol

Le produit attendu est obtenu selon le procédé décrit dans le stade A de l'exemple 3 à partir du composé décrit dans l'exemple 36.

### Stade B : (2R*,4S*,6R*)-6-Méthyl-2-(3-pyridyl)-4-pipéridinol, dioxalate

A 10 mmoles du composé obtenu dans le stade précédent en solution dans l'isopropanol sont ajoutées 22 mmoles d'acide oxalique. Après 1 h d'agitation à température ambiante, les solvants sont évaporés pour conduire au produit attendu sous forme de cristaux.

| Microanalyse élémentaire : | | | |
|---|---|---|---|
| | %C | %H | %N |
| calculé | 48,39 | 5,41 | 7,52 |
| trouvé | 48,26 | 5,46 | 7,55 |

### EXEMPLE 38 : (±)-N-Acétyl-6-méthyl-2-(3-pyridyl)-2,3-dihydro-4(1H)-pyridinone

Le produit attendu est obtenu selon le procédé décrit dans l'exemple 2 à partir du composé décrit dans l'exemple 36, en remplaçant l'iodométhane par le chlorure d'acétyle.

### EXEMPLE 39 : (2R*,4S*,6R*)-N-Acétyl-6-méthyl-2-(3-pyridyl)-4-pipéridinol

Le produit attendu est obtenu selon le procédé décrit dans le stade A de l'exemple 3 à partir du composé décrit dans l'exemple 38.

### EXEMPLE 40 : (2R*,4R*,6R*)-N-Acétyl-4-chloro-6-méthyl-2-(3-pyridyl)-pipéridine

### Stade A : (2R*,4S*,6R*)-N-Acétyl-6-méthyl-2-(3-pyridyl)-4-pipéridinone

Le produit attendu est obtenu selon le procédé décrit dans le stade A de l'exemple 12 à partir du composé décrit dans l'exemple 39.

### Stade B : (2R*,4R*,6R*)-N-Acétyl-4-chloro-6-méthyl-2-(3-pyridyl)-pipéridine

Le produit attendu est obtenu selon le procédé décrit dans le stade A de l'exemple 13 à partir du composé obtenu dans le stade précédent.

### EXEMPLE 41 : (2R*,4S*,6R*)-N-Acétyl-6-méthyl-4-méthylthio-2-(3-pyridyl)-pipéridine

Le produit attendu est obtenu selon le procédé décrit dans le stade A de l'exemple 14 à partir du composé décrit dans l'exemple 40 et de méthanethiol.

### EXEMPLE 42 : (2R*,4S*,6R*)-N-Acétyl-4-méthoxycarbonyloxy-6-méthyl-2-(3-pyridyl)-pipéridine

Le produit attendu est obtenu selon le procédé décrit dans l'exemple 4 à partir du composé décrit dans l'exemple 39, en remplaçant l'iodométhane par le chloroformiate de méthyle.

### EXEMPLE 43 : (±)-N-Benzyl-6-propyl-2-(3-pyridyl)-2,3-dihydro-4(1H)-pyridinone

### Stade A : (±)-6-Propyl-2-(3-pyridyl)-2,3-dihydro-4(1H)-pyridinone

Le produit attendu est obtenu selon le procédé décrit dans l'exemple 1 à partir de nicotinaldéhyde et de nonane-4,6-dione.

### Stade B : (±)-N-Benzyl-6-propyl-2-(3-pyridyl)-2,3-dihydro-4(1H)-pyridinone

Le produit attendu est obtenu selon le procédé décrit dans l'exemple 2 à partir du composé obtenu dans le stade précédent, en remplaçant l'iodométhane par le chlorure de benzyle.

### EXEMPLE 44 : (±)-N-Benzyl-6-propyl-2-(3-pyridyl)-2,3-dihydro-4(1H)-pyridinone O-[2-(diméthylamino)-éthyl]-oxime

Le produit attendu est obtenu selon le procédé décrit dans l'exemple 15 à partir du composé décrit dans l'exemple 43, en remplaçant le chlorhydrate d'hydroxylamine par la 2-(aminooxy)-N,N-diméthyléthanamine.

### ETUDE PHARMACOLOGIQUE DES DERIVES DE L'INVENTION

### EXEMPLE 45 : Locomotion chez le Rat Wistar

Les effets sur la motricité des composés de la présente invention ont été évalués chez le rat Wistar adulte mâle. Des rats Wistar (180-200 g) ont été traités (voie intrapéritonéale) par les produits sous étude ou leurs véhicules (2 ml/kg). Trente minutes après traitement pharmacologique, les animaux étaient placés dans un open-field (40x40x60 cm), situé dans une salle d'expérimentation à environnement contrôlé. La motricité des animaux a été évaluée par la mesure de la distance parcourue (cm) dans cet open-field pendant 30 minutes. Cette mesure était effectuée automatiquement par un système de vidéo-surveillance couplé à un micro-ordinateur (système Vidéotrack, View-Point, France). Les résultats ont été exprimés en moyennes plus ou moins les erreurs standard sur les moyennes, et les comparaisons inter-lots ont été réalisées par un test d'analyse de variance à un facteur suivi le cas échéant par un test de Dunnett.

Les résultats montrent que les composés de l'invention sont dépourvus d'activité sur la locomotion chez le rat jusqu'à la dose de 10 mg/kg.

### EXEMPLE 46 : Température corporelle chez la souris NMRI

Les effets sur la température corporelle des composés de la présente intervention ont été évalués chez la souris NMRI adulte mâle. La température rectale des souris (18-20 g) a été mesurée juste avant traitement pharmacologique (voie intrapéritonéale) par les produits sous étude ou leurs véhicules (20 mg/kg). Les souris étaient ensuite placées dans des cages individuelles (10x10x10 cm) et leur température rectale a été mesurée toutes les 30 minutes pendant les 2 heures qui ont suivi le traitement. Les valeurs étaient les moyennes (°C) plus ou moins les erreurs standards sur les moyennes, et les comparaisons inter-lots ont été réalisées par un test d'analyse de variance à un facteur suivi le cas échéant par un test de Dunnett.

Les résultats montrent que les composés de l'invention sont dépourvus d'activité hypothermisante pour des doses allant jusqu'à 20 mg/kg.

### EXEMPLE 47 : Torsions abdominales induites à la phényl-p-benzoquinone (PBQ) chez la souris NMRI

L'administration intrapéritonéale d'une solution alcoolique de PBQ provoque des crampes abdominales chez la Souris (SIEGMUND et coll., Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (COLLIER et coll., Brit. J. Pharmacol. Chem., 1968, 32, 295-310). A t=0 min., les animaux sont pesés et le produit étudié est administré par voie IP. Un groupe d'animaux témoins reçoit le solvant du produit. A t=30 min., une solution alcoolique de PBQ (0,2 %) est administrée par voie IP sous un volume de 0,25 ml/souris. Immédiatement après l'administration de la PBQ, les animaux sont placés dans des cylindres en plexiglass (L=19,5 cm ; D.I.=5 cm). De t=35 min. à t=45 min., la réaction des animaux est observée et l'expérimentateur note le nombre total de crampes abdominales par animal. Le tableau ci-dessous donne le pourcentage d'inhibition du nombre de crampes abdominales mesuré chez les animaux témoins, à la dose active du composé étudié.
Les résultats obtenus montrent que les composés de l'invention sont pourvus de propriétés antalgiques.

| Exemple | Dose (mg/kg) | Inhibition (%) |
|---|---|---|
| 1 | 10 | 51 |
| 25 | 20 | 69 |
| 26 | 10 | 55 |
| 30 | 20 | 51 |

### EXEMPLE 48 : Reconnaissance sociale chez le rat Wistar

Initialement décrit en 1982 par THOR et HOLLOWAY, (J. Comp. Physiol., 1982, 96, 1000-1006), le test de la reconnaissance sociale a été ensuite proposé par différents auteurs (DANTZER et coll., Psychopharmacology, 1987, 91, 363-368 ; PERIO et coll., Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester (voie intrapéritonéale) et, 2 heures plus tard, est remis en présence (5 minutes) du jeune rat. Le comportement de reconnaissance sociale est alors à nouveau observé et la durée en est mesurée. Le tableau ci-dessous donne la différence (T₂-T₁), exprimée en secondes, des temps de "reconnaissance" des 2 rencontres.

Les résultats obtenus montrent que les composés de l'invention augmentent la mémorisation de façon très importante, et à faible dose.

| Exemple | Dose (mg/kg) | T₂-T₁ (s) |
|---|---|---|
| 1 | 3 | -36 |
| 21 | 3 | -33 |
| 24 | 3 | -32 |
| 36 | 3 | -36 |

### EXEMPLE 49 : Reconnaissance d'objet chez le rat Wistar

Le test de reconnaissance d'objet chez le rat Wistar a été initialement développé par ENNACEUR et DELACOUR (Behav. Brain Res., 1988, 31, 47-59). Ce test est basé sur l'activité exploratoire spontanée de l'animal et possède les caractéristiques de la mémoire épisodique chez l'homme. Sensible au vieillissement (SCALI et coll., Eur. J. Pharmacol., 1997, 325, 173-180), ainsi qu'aux dysfonctionnements cholinergiques (BARTOLINI et coll., Pharm. Biochem. Behav. 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie IP ou p.o. 30 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté. Le tableau ci-dessous donne la différence Delta, exprimée en secondes, des temps d'exploration des deux objets.

Les résultats obtenus montrent que les composés de l'invention augmentent la mémorisation de façon importante, à très faible dose, que ce soit par voie i.p. ou p.o.

| Exemple | Dose (mg/kg) | Delta (s) | Voie |
|---|---|---|---|
| 1 | 0,3 | 7,5 | i.p. |
| | 0,3 | 9,95 | p.o. |
| 18 | 1 | 6,2 | p.o. |
| 22 | 0,3 | 9,98 | i.p. |
| | 3 | 5,93 | p.o. |
| 24 | 3 | 7,55 | i.p. |
| 27 | 3 | 6,68 | i.p. |

### EXEMPLE 50 : Composition pharmaceutique

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 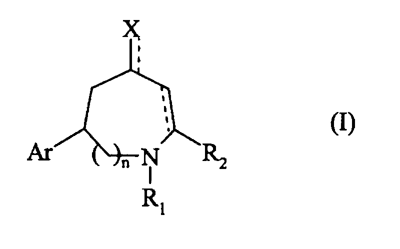 dans laquelle :
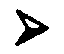 n représente 0 ou 1,
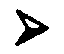 R₁ représente un atome d'hydrogène,
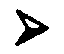 R₂ représente un groupement alkyle (C₁-C₆) linéaire ou ramifié,
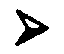 X représente un atome d'oxygène ou un groupement OR₃,
* R₃ représente un atome d'hydrogène,
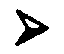 ---- représente une liaison simple ou double, étant entendu que la valence des atomes est respectée,
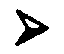 Ar représente un groupement aryle ou un groupement hétéroaryle,
leurs isomères ainsi que leurs sels d'addition à un acide pharmaceutiquement acceptable,
à la condition que les composés de formule (I) soient différents :
- de la 6-méthyl-2-phényl-2,3-dihydro-4-pyridinone,
- de la 2-méthyl-6-phényl-4-pipéridinone,
- des N-benzyl-2-(R'₂)-6-phényl-4-pipéridinones dans lesquelles R'₂ représente le groupement méthyle, éthyle, propyle ou isopropyle,
- et des 2-(R"₂)-6-phényl-4-pipéridinols dans lesquels R"₂ représente le groupement isopropyle ou butyle,
étant entendu que par groupement aryle, on entend phényle éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié).
et par groupement hétéroaryle, on entend thiényle ou pyridyle éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, trihalogénométhyle, et amino (substitué éventuellement par un ou plusieurs groupements alkyle (C₁-C₆) linéaire ou ramifié).

2. Composé de formule (I) selon la revendication 1 tel que n représente 0.

3. Composé de formule (I) selon l'une quelconque des revendications 1 ou 2 tel que Ar représente le groupement phényle éventuellement substitué.

4. Composé de formule (I) selon l'une quelconque des revendications 1 ou 2 tel que Ar représente le groupement thiényle éventuellement substitué.

5. Composé de formule (I) selon l'une quelconque des revendications 1 ou 2 tel que Ar représente le groupement pyridyle éventuellement substitué.

6. Composé de formule (I) selon la revendication 1 qui est la (±)-2-(3-chlorophényl)-6-méthyl-2,3-dihydro-4(1H)-pyridinone, ses isomères ainsi que ses sels d'addition à un acide pharmaceutiquement acceptable.

7. Composé de formule (I) selon la revendication 1 qui est le (2R*,4S*,6R*)-2-phényl-6-méthyl-4-pipéridinol, ses isomères ainsi que ses sels d'addition à un acide pharmaceutiquement acceptable.

8. Procédé de préparation des composés de formule (I) selon la revendication 1 caractérisé en en ce que l'on met en réaction un composé de formule (II) : 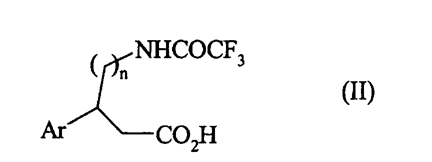 dans laquelle Ar et n ont la même signification que dans la formule (I),
avec du chlorure de thionyle, pour conduire au chlorure d'acide correspondant que l'on fait réagir avec un composé de formule (III) : 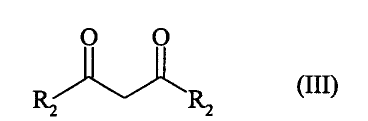 dans laquelle R₂ a la même signification que dans la formule (I),
en présence de triiodure de samarium,
pour conduire après déprotection par un acide HA au composé de formule (IV) : 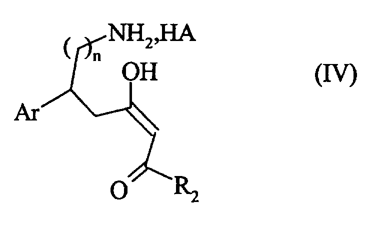 dans laquelle Ar, n et R₂ ont la même signification que précédemment, et HA représente un acide donneur de proton,
composé de formule (IV), que l'on fait ensuite réagir en milieu basique pour conduire au composé de formule (Ia), cas particulier des composés de formule (I) : 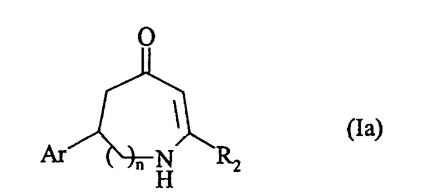 dans laquelle Ar, n et R₂ ont la même signification que précédemment,
composé de formule (la) que l'on transforme, si on le désire,
- soit par réduction partielle à l'aide d'un agent réducteur approprié, suivie, lorsqu'on le souhaite, d'une alkylation, acylation ou estérification de la fonction hydroxy, pour conduire au composé de formule (Ic), cas particulier des composés de formule (I) : 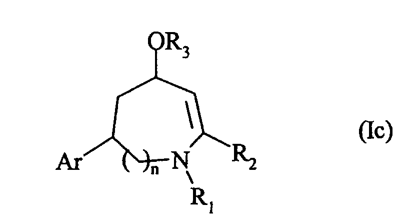 dans laquelle Ar, n et R₂ ont la même signification que précédemment, et R₁ et R₃ ont la même signification que dans la formule (I),
- soit par réduction totale à l'aide d'un agent réducteur approprié, pour conduire au composé de formule (Id), cas particulier des composés de formule (I) : 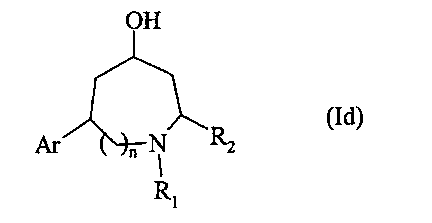
dans laquelle Ar, n, R₁ et R₂ ont la même signification que précédemment,
composé de formule (Id) que l'on fait réagir, si on le souhaite,
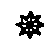 par une réaction d'oxydation à l'aide d'un agent oxydant approprié, pour conduire au composé de formule (If), cas particulier des composés de formule (I) : 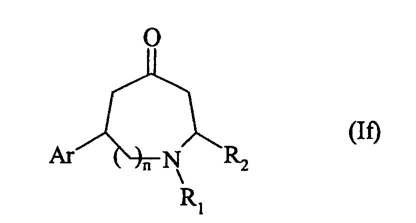 dans laquelle Ar, n, R₁ et R₂ ont la même signification que précédemment,
composés de formule (Ia), (Ic), (Id), ou (If) qui constituent l'ensemble des composés de formule (I), que l'on purifie, le cas échéant, selon une technique classique de purification, dont on sépare, si on le souhaite, les isomères selon une technique classique de séparation, et que l'on transforme, si on le souhaite, en leurs sels d'addition à un acide pharmaceutiquement acceptable.

9. Composition pharmaceutique contenant comme principe actif un composé selon l'une quelconque des revendications 1 à 7, seul ou en combinaison avec un ou plusieurs véhicules inertes, non toxiques et pharmaceutiquement acceptables.

10. Composition pharmaceutique selon la revendication 9 utile en tant que facilitateur mnémocognitif.

11. Composition pharmaceutique selon la revendication 9 utile en tant qu'antalgique.

## Patentansprüche

1. Verbindungen der Formel (I): 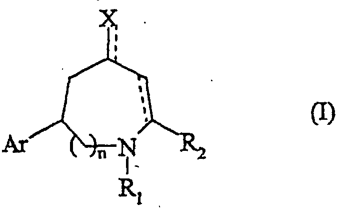 in der:
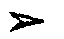 n 0 oder 1 bedeutet,
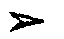 R₁ ein Wasserstoffatom bedeutet,
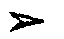 R₂ eine geradkettige oder verzweigte (C₁-C₆)-Alkylgruppe bedeutet,
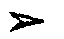 X ein Sauerstoffatom oder eine Gruppe OR₃ bedeutet,
* worin R₃ ein Wasserstoffatom darstellt,
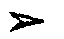 ---- eine Einfachbindung oder eine Doppelbindung bedeutet, mit der Maßgabe, daß die Wertigkeit der Atome berücksichtigt ist, und
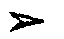 Ar eine Arylgruppe oder eine Heteroarylgruppe bedeutet,
deren Isomere sowie deren Additionssalze mit einer pharmazeutisch annehmbaren Säure,
mit der Maßgabe, daß die Verbindungen der Formel (I) verschieden sind von:
- 6-Methyl-2-phenyl-2,3-dihydro-4-pyridinon,
- 2-Methyl-6-phenyl-4-piperidinon,
- N-Benzyl-2-(R'₂)-6-phenyl-4-piperidinonen, worin R'₂ die Methyl-, Ethyl-, Propyl- oder Isopropylgruppe bedeutet, und
- 2-(R"₂)-6-Phenyl-4-piperidinolen, worin R"₂ die Isopropyl- oder Butylgruppe darstellt,
mit der Maßgabe, daß man unter einer Arylgruppe eine Phenylgruppe versteht, die gegebenenfalls durch eine oder mehrere gleichartige oder verschiedenartige Gruppen ausgewählt aus Halogen, geradkettigem oder verzweigtem (C₁-C₆)-Alkyl, Hydroxy, geradkettigem oder verzweigtem (C₁-C₆)-Alkoxy, Trihalogenmethyl und Amino (gegebenenfalls substituiert durch eine oder mehrere geradkettige oder verzweigte (C₁-C₆)-Alkylgruppen) substituiert ist,
und unter einer Heteroarylgruppe eine Thienyl- oder Pyridiylgruppe versteht, die gegebenenfalls durch eine oder mehrere gleichartige oder verschiedenartige Gruppen ausgewählt aus Halogen, geradkettigem oder verzweigtem (C₁-C₆)-Alkyl, Hydroxy, geradkettigem oder verzweigtem (C₁-C₆)-Alkoxy, Trihalogenmethyl und Amino (gegebenenfalls substituiert durch eine oder mehrere geradkettige oder verzweigte (C₁-C₆)-Alkylgruppen) substituiert sind.

2. Verbindung der Formel (I) nach Anspruch 1, worin n den Wert 0 besitzt.

3. Verbindung der Formel (I) nach einem der Ansprüche 1 oder 2, worin Ar eine gegebenenfalls substituierte Phenylgruppe bedeutet.

4. Verbindung der Formel (I) nach einem der Ansprüche 1 oder 2, worin Ar eine gegebenenfalls substituierte Thienylgruppe bedeutet.

5. Verbindung der Formel (I) nach einem der Ansprüche 1 oder 2, worin Ar eine gegebenenfalls substituierte Pyridylgruppe bedeutet.

6. Verbindung der Formel (I) nach Anspruch 1, nämlich (±)-2-(3-Chlorphenyl)-6-methyl-2,3-dihydro-4(1H)-pyridinon, dessen Isomere sowie dessen Additionssalze mit einer pharmazeutisch annehmbaren Säure.

7. Verbindung der Formel (I) nach Anspruch 1, nämlich (2R*,4S*,6R*)-2-Phenyl-6-methyl-4-piperidinol, dessen Isomere sowie dessen Additionssalze mit einer pharmazeutisch annehmbaren Säure.

8. Verfahren zur Herstellung der Verbindungen der Formel (I) nach Anspruch 1, **dadurch gekennzeichnet, daß** man eine Verbindung der Formel (II): 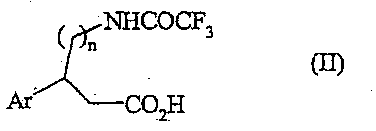 in der Ar und n die bezüglich der Formel (I) angegebenen Bedeutungen besitzen,
mit Thionylchlorid umsetzt zur Bildung des entsprechenden Säurechlorids, welches man in Gegenwart von Samariumtriiodid mit einer Verbindung der Formel (III): 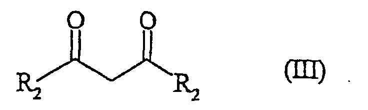 in der R₂ die bezüglich der Formel (I) angegebenen Bedeutungen besitzt, umsetzt, so daß man nach der Abspaltung der Schutzgruppe mit einer Säure HA die Verbindung der Formel (IV) erhält: 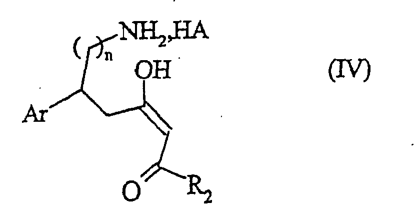 in der Ar, n und R₂ die oben angegebenen Bedeutungen besitzen und HA eine Protonendonor-Säure darstellt,
welche Verbindung der Formel (IV) man anschließend in basischem Medium umsetzt zur Bildung der Verbindung der Formel (Ia), einem Sonderfall der Verbindungen der Formel (I): 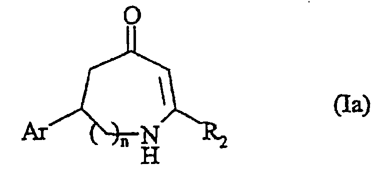 in der Ar, n und R₂ die oben angegebenen Bedeutungen besitzen, welche Verbindung der Formel (Ia) man gewünschtenfalls
- entweder durch teilweise Reduktion mit Hilfe eines geeigneten Reduktionsmittels, gegebenenfalls gefolgt von einer Alkylierung, Acylierung oder Veresterung der Hydroxygruppe in die Verbindung der Formel (Ic) umwandelt, einem Sonderfall der Verbindungen der Formel (I): 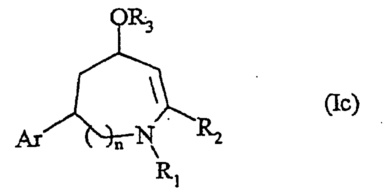 in der Ar, n und R₂ die oben angegebenen Bedeutungen besitzen und R₁ und R₃ die bezüglich der Formel (I) angegebenen Bedeutungen besitzen,
- oder durch vollständige Reduktion mit Hilfe eines geeigneten Reduktionsmittels in die Verbindung der Formel (Id) umwandelt, einem Sonderfall der Verbindungen der Formel (I): 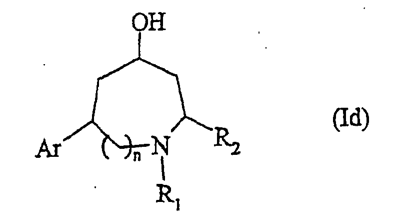
in der Ar, n, R₁ und R₂ die oben angegebenen Bedeutungen besitzen, welche Verbindung der Formel (Id) man gewünschtenfalls
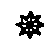 durch eine Oxidationsreaktion mit Hilfe eines geeigneten Oxidationsmittels zu der Verbindung der Formel (If) umsetzt, einem Sonderfall der Verbindungen der Formel (I): 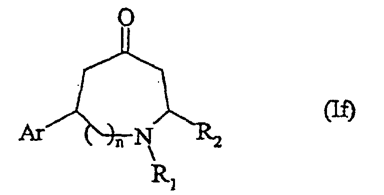
in der Ar, n, R₁ und R₂ die oben angegebenen Bedeutungen besitzen, welche Verbindungen der Formeln (Ia), (Ic), (Id) und (If), welche die Gesamtheit der Verbindungen der Formel (I) bilden, man gegebenenfalls mit Hilfe einer klassischen Reinigungsmethode reinigt, gewünschtenfalls mit Hilfe einer klassischen Trennmethode in ihre Isomeren auftrennt und gewünschtenfalls in ihre Additionssalze mit einer pharmazeutisch annehmbaren Säure überführt.

9. Pharmazeutische Zubereitung enthaltend als Wirkstoff eine Verbindung nach einem der Ansprüche 1 bis 7 allein oder in Kombination mit einem oder mehreren inerten, nichttoxischen und pharmazeutisch annehmbaren Trägermaterialien.

10. Pharmazeutische Zubereitung nach Anspruch 9 nützlich als Mittel zur Erleichterung des mnemokognitiven Verhaltens.

11. Pharmazeutische Zubereitung nach Anspruch 9 nützlich als Analgetikum.

## Claims

1. Compounds of formula (I) : 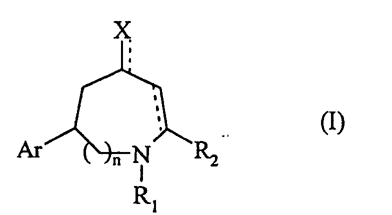 wherein:
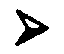 n represents 0 or 1,
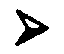 R₁ represents a hydrogen atom,
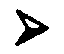 R₂ represents a linear or branched (C₁-C₆)alkyl group,
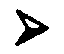 X represents an oxygen atom or a group OR₃,
* R₃ represents a hydrogen atom,
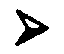 ---- represents a single or double bond, it being understood that the valency of the atoms is respected,
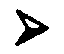 Ar represents an aryl group or a heteroaryl group,
their isomers and addition salts thereof with a pharmaceutically acceptable acid,
with the proviso that the compounds of formula (I) are other than :
- 6-methyl-2-phenyl-2,3-dihydro-4-pyridinone,
- 2-methyl-6-phenyl-4-piperidinone,
- N-benzyl-2-(R'₂)-6-phenyl-4-piperidinones wherein R'₂ represents a methyl, ethyl propyl or isopropyl group,
- and 2-(R"₂)-6-phenyl-4-piperidinols wherein R"₂ represents an isopropyl or butyl group,
an aryl group being understood to be phenyl optionally substituted by one or more identical or different groups selected from halogen, linear or branched (C₁-C₆)alkyl, hydroxy, linear or branched (C₁-C₆)alkoxy, trihalomethyl and amino (optionally substituted by one or more linear or branched (C₁-C₆)alkyl groups),
and a heteroaryl group being understood to be thienyl or pyridyl each optionally substituted by one or more identical or different groups selected from halogen, linear or branched (C₁-C₆)alkyl, hydroxy, linear or branched (C₁-C₆)alkoxy, trihalomethyl and amino (optionally substituted by one or more linear or branched (C₁-C₆)alkyl groups).

2. Compound of formula (I) according to claim 1, in which n represents 0.

3. Compound of formula (I) according to either claim 1 or claim 2, in which Ar represents an optionally substituted phenyl group.

4. Compound of formula (I) according to either claim 1 or claim 2, in which Ar represents an optionally substituted thienyl group.

5. Compound of formula (I) according to either claim 1 or claim 2, in which Ar represents an optionally substituted pyridyl group.

6. Compound of formula (I) according to claim 1, which is (+)-2-(3-chlorophenyl)-6 methyl-2,3-dihydro-4(1H)-pyridinone, its isomers and addition salts thereof with a pharmaceutically acceptable acid.

7. Compound of formula (I) according to claim 1, which is (2R*,4S*,6R*)-2-phenyl 6-methyl-4-piperidinol, its isomers and addition salts thereof with a pharmaceutically acceptable acid.

8. Process for the preparation of-compounds of formula (I) according to claim 1, **characterised in that** a compound of formula (II) : 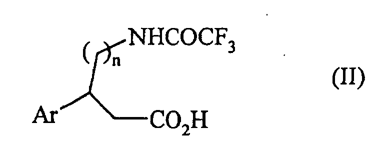 wherein Ar and n are as defined for formula (I),
is reacted with thionyl chloride to yield the corresponding acid chloride, which is reacted with a compound of formula (III) : 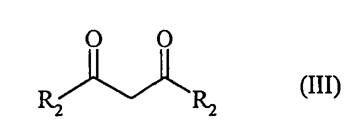 wherein R₂ is as defined for formula (I),
in the presence of samarium triiodide,
to yield, after deprotection by an acid HA, the compound of formula (IV) : 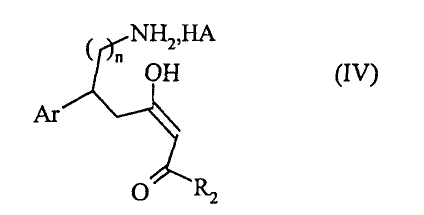 wherein Ar, n and R₂ are as defined hereinbefore and HA represents a proton donor acid,
which compound of formula (IV) is then reacted in a basic medium to yield the compound of formula (Ia), a particular case of the compounds of formula (I): 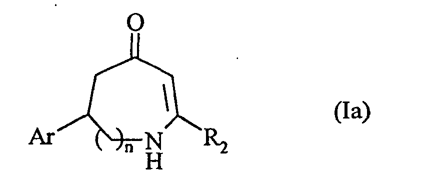 wherein Ar, n and R₂ are as defined hereinbefore,
which compound of formula (Ia) is converted, if desired,
- either by partial reduction with the aid of an appropriate reducing agent, followed, if desired, by alkylation, acylation or esterification of the hydroxy function to yield the compound of formula (Ic), a particular case of the compounds of formula (I) : 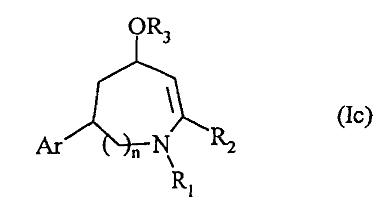 wherein Ar, n and R₂ are as defined hereinbefore and R₁ and R₃ are as defined for formula (I),
- or by complete reduction with the aid of an appropriate reducing agent to yield the compound of formula (Id), a particular case of the compounds of formula (I) : 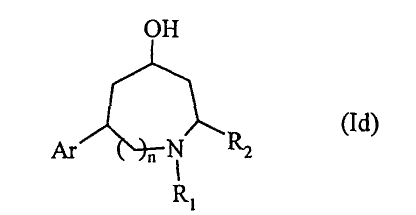
wherein Ar, n, R₁ and R₂ are as defined hereinbefore,
which compound of formula (Id) is reacted, if desired,
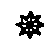 in an oxidation reaction using an appropriate oxidising agent to yield the compound of formula (If), a particular case of the compounds of formula (I) : 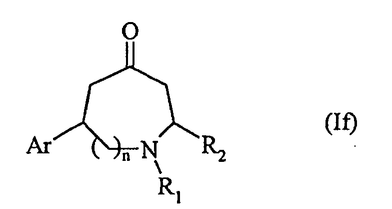 wherein Ar, n, R₁ and R₂ are as defined hereinbefore,
the compounds of formulae (Ia), (Ic), (Id) and (If) constituting the totality of the compounds of formula (I), which are purified, if necessary, according to a conventional purification technique, are separated, if desired, into their isomers according to a conventional separation technique and are converted, if desired, into their addition salts with a pharmaceutically acceptable acid.

9. Pharmaceutical composition comprising as active ingredient a compound according to any one of claims 1 to 7, alone or in combination with one or more inert, nontoxic, pharmaceutically acceptable carriers.

10. Pharmaceutical composition according to claim 9 for use as a facilitator of memory and cognition.

11. Pharmaceutical composition according to claim 9 for use as an antalgic agent.
